(19) Europäisches Patentamt
European Patent Office
Office européen des brevets

(11) **EP 3 358 060 B1**

(12) **EUROPEAN PATENT SPECIFICATION**

(45) Date of publication and mention of the grant of the patent:
**30.06.2021 Bulletin 2021/26**

(21) Application number: **16851201.0**

(22) Date of filing: **15.09.2016**

(51) Int Cl.:
*A44B 18/00* (2006.01)   *D04H 1/559* (2012.01)
*D04H 11/08* (2006.01)   *D06C 23/04* (2006.01)
*A61F 13/62* (2006.01)   *B29C 65/08* (2006.01)
*B29C 65/78* (2006.01)   *B29C 65/00* (2006.01)
*B32B 5/24* (2006.01)   *D06H 5/00* (2006.01)

(86) International application number:
**PCT/JP2016/077307**

(87) International publication number:
**WO 2017/057027 (06.04.2017 Gazette 2017/14)**

(54) **LAYERED ARTICLE**

MEHRLAGIGER ARTIKEL

ARTICLE EN COUCHES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.09.2015   JP 2015190789**
**06.09.2016   JP 2016173303**

(43) Date of publication of application:
**08.08.2018   Bulletin 2018/32**

(73) Proprietor: **Nitto Denko Corporation**
**Ibaraki-shi, Osaka 567-8680 (JP)**

(72) Inventors:
• **UCHIDA, Shou**
**Ibaraki-shi**
**Osaka 567-8680 (JP)**
• **IKISHIMA, Shinsuke**
**Ibaraki-shi**
**Osaka 567-8680 (JP)**
• **TAKEDA, Kohei**
**Ibaraki-shi**
**Osaka 567-8680 (JP)**
• **NAKAGAWA, Muneshige**
**Ibaraki-shi**
**Osaka 567-8680 (JP)**

(74) Representative: **Müller-Boré & Partner**
**Patentanwälte PartG mbB**
**Friedenheimer Brücke 21**
**80639 München (DE)**

(56) References cited:
EP-A1- 3 311 687      EP-A1- 3 311 688
WO-A1-2007/114387      JP-A- H0 363 683
JP-A- H10 280 267      JP-A- 2004 176 191
JP-A- 2011 135 943      JP-A- 2011 135 985
JP-A- 2012 167 412      JP-A- 2013 209 787
JP-A- 2014 224 330      US-A1- 2006 019 572

**Description**

The present invention relates to a layered article. The present invention also relates to a hook-and- loop fastener including the layered article of the present invention. Document JP H10 280267 discloses a layered article according to the preamble of claim 1.

Layered articles are proposed for materials for articles such as sanitary articles, for example, diapers and masks (see, for example, Patent Literatures 1 and 2).

In recent years, non-woven fabrics have been adopted for many of the layered articles to be used for sanitary articles (inparticular, disposable diapers, supporters, masks, and the like).

However, the related-art layered articles adopting the non-woven fabrics each involve a problem in that its flexibility is not sufficient and hence a satisfactory touch feeling cannot be achieved.

[PTL 1] JP 2009-527315 A
[PTL 2] JP 2010-125337 A
[PTL 3] JPH10-280267 A describes a flexible spun-bonded nonwoven fabric.
[PTL 4] JPH03-63683 A discloses a cleaning sheet.
[PTL 5] JP 2012-167412 A relates to a water-liftable fiber structure.
[PTL 6] JP 2004-176191 A describes a fabric, a method for producing the same and an air-permeable member.
[PTL 7] JP 2014-224330 A discloses a molding processing sheet and a method for producing a molding processing sheet.
[PTL 8] EP 3 311 687 A1 describes a female member for a touch fastener.
[PTL 9] EP 3 311 688 A1 also discloses a female member for a touch fastener.
[PTL 10] US 2006/019572 A1 relates to a hook and loop fastener device.
[PTL 11] JP 2011-135985 A describes a disposable diaper.

The present invention has been made to solve the conventional problem, and an object of the present invention is to provide a layered article including at least one layer including a non-woven fabric of fiber, the layered article having sufficient flexibility and being capable of achieving a satisfactory touch feeling. Another object of the present invention is to provide a hook-and-loop fastener including the layered article of the present invention.

A layered article according to the present invention is a layered article, including at least one layer including a non-woven fabric of fiber, in which:

the layered article has a fused portion; and
the layered article has an index A, which is represented by the equation (1), of $0.75 \times 10^{-6}$ m$^3$/g or less:

$$A = T/G \qquad (1)$$

where T represents a thickness (unit: m) of the fused portion and G represents a basis weight (unit: g/m$^2$), wherein T is from $5 \times 10^{-6}$ m to $28 \times 10^{-6}$ m.

In one embodiment, the indexA is from $0.20 \times 10^{-6}$ m$^3$/g to $0.72 \times 10^{-6}$ m$^3$/g.

In one embodiment, the G is 50 g/m$^2$ or less.

In one embodiment, the G is 48 g/m$^2$ or less.

In one embodiment, the fused portion includes a fused portion of pieces of the fiber.

In one embodiment, the number of holes each having a diameter of 100 $\mu$m or more present in the fused portion is 10 holes/m$^2$ or less.

In one embodiment, the fused portion has an embossed pattern.

In one embodiment, the layered article includes a hook- and-loop fastener female member.

In one embodiment, the layered article further includes: an engaging layer engageable with a hook-and-loop fastener male member; and a physical property layer configured to hold the engaging layer.

In one embodiment, a sum of a basis weight of a non-woven fabric in the engaging layer and a basis weight of a non-woven fabric in the physical property layer is 60 g/m$^2$ or less.

In one embodiment, a surface of fiber forming a non-woven fabric included in the engaging layer and a surface of the physical property layer on the engaging layer side contain the same kind of polymer.

In one embodiment, a hook-and-loop fastener of the present invention includes: the above-mentioned layered article; and a hook-and-loop fastener male member configured to engage with the layered article.

According to the present invention, the layered article including at least one layer including a non-woven fabric of fiber, the layered article having sufficient flexibility and being capable of achieving a satisfactory touch feeling can be provided. The hook-and-loop fastener including such layered article can also be provided.

FIG. 1 is a schematic plan view of an example of an embossed pattern of an arc shape.
FIG. 2 is a schematic sectional view for illustrating an example of a preferred method of producing a layered article of the present invention.

<<1. Layered Article of the Present Invention>>

A layered article of the present invention is a layered article including at least one layer including a non-woven fabric of fiber. The number of the layers each including the non-woven fabric of fiber of the layered article of the present invention is preferably from 1 to 10, more preferably from 1 to 7, still more preferably from 1 to 5, particularly preferably from 1 to 3, most preferably 2 or 3. When the number of the layers each including the non-woven fabric of fiber of the layered article of the present invention falls within the above-mentioned range, the layered article has more sufficient flexibility and can achieve a more satisfactory touch feeling.

The layered article of the present invention may include any appropriate other layer as long as the layered article includes at least one layer including the non-woven fabric of fiber, and the effect of the present invention is not impaired.

The layered article of the present invention has a fused portion. When the layered article of the present invention has the fused portion, the layered article of the present invention has more sufficient flexibility and can achieve a more satisfactory touch feeling.

The fused portion preferably includes a fused portion of pieces of the fiber of the layer including the non-woven fabric of fiber.

In the fused portion, the number of holes each having a diameter of 100 $\mu$m or more present in the fused portion is preferably 10 holes/m$^2$ or less, more preferably 5 holes/m$^2$ or less, still more preferably 3 holes/m$^2$ or less, particularly preferably 0 holes/m$^2$. When the number of the holes each having a diameter of 100 $\mu$m or more present in the fused portion falls within the above-mentioned range, the external appearance of the layered article is improved and its breakage starting from the holes hardly occurs.

The fused portion preferably has an embossed pattern. Such embossed pattern is preferably formed by embossing treatment. Specific examples of the embossed pattern include a continuous grid shape, a discontinuous grid shape, a continuous curve shape, a discontinuous curve shape, a continuous zigzag shape, a discontinuous zigzag shape, a continuous linear shape, a discontinuous linear shape, a circle shape, an ellipse shape, a hollow circle shape, a hollow ellipse shape, an arc shape, and a hollow arc shape.

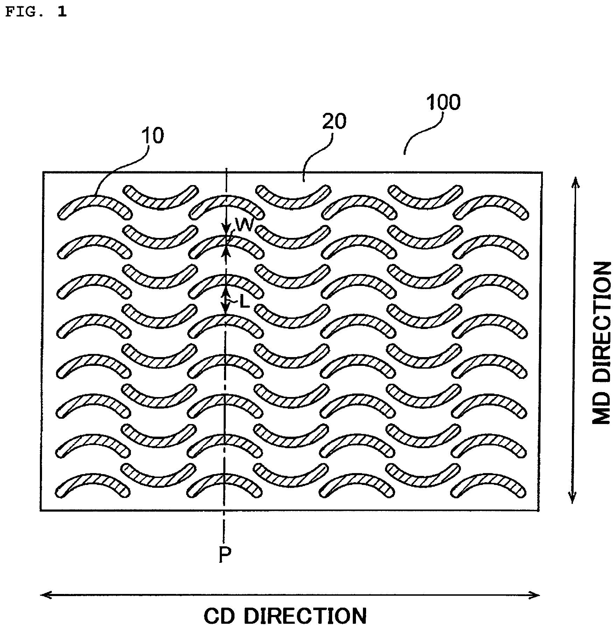

The embossed pattern is preferably a discontinuous embossed pattern, more preferably an embossed pattern of an arc shape, because the effect of the present invention can be more effectively expressed. A schematic plan view of an example of the embossed pattern of an arc shape is illustrated in FIG. 1. In FIG. 1, a fused portion 100 has a plurality of embossments 10 forming an embossed pattern of an arc shape. In FIG. 1, the fused portion 100 has a region 20 free of the embossed pattern. In the embossed pattern of an arc shape, it is preferred that individual embossments be embossments having no "corners".

The embossment width of each of the plurality of embossments forming the embossed pattern is preferably from 0.1 mm to 3.0 mm, more preferably from 0.3 mm to 2.0 mm, still more preferably from 0.3 mm to 1.5 mm, particularly preferably from 0.5 mm to 1.5 mm, most preferably from 0.5 mm to 1.0 mm. When the embossment width falls within the above-mentioned range, the layered article of the present invention has more sufficient flexibility and can achieve a more satisfactory touch feeling. The embossment width refers to, for example, a width W of each of the embossments 10 in an MD direction as illustrated in FIG. 1.

The distance between two adj acent embossments in the plurality of embossments forming the embossed pattern on any line in the MD direction is preferably 10 mm or less, more preferably from 1 mm to 10 mm, still more preferably from 1.5 mm to 9 mm, particularly preferably from 2 mm to 8 mm, most preferably from 2.5 mm to 7 mm. When the distance between two adjacent embossments in the plurality of embossments forming the embossed pattern on any line in the MD direction falls within the above-mentioned range, the layered article of the present invention has more sufficient flexibility and can achieve a more satisfactory touch feeling. The distance between two adjacent embossments in the plurality of embossments forming the embossed pattern on a line in the MD direction is, for example, a distance L between two adjacent embossments on a line P in the MD direction illustrated in FIG. 1 (which may be a line in the MD direction at any position in a CD direction).

The depth of each of the embossments is preferably from 0.1 mm to 2.0 mm, more preferably from 0.2 mm to 1.8 mm, still more preferably from 0.3 mm to 1.5 mm, particularly preferably from 0.5 mm to 1.5 mm, most preferably from 0.7 mm to 1.2 mm. When the depth of each of the embossments falls within the above-mentioned range, the layered article of the present invention has more sufficient flexibility and can achieve a more satisfactory touch feeling.

The ratio of the area of a fused portion formed by the embossed pattern to the area of the entire surface of the layered article of the present invention (hereinafter sometimes referred to as "embossing-fused area ratio") is preferably 50% or less, more preferably from 1% to 40%, still more preferably from 5% to 35%, particularly preferably from 10% to 30%, most preferably from 15% to 25%. When the embossing-fused area ratio falls within the above-mentioned range, the layered article of the present invention has more sufficient flexibility and can achieve a more satisfactory touch feeling.

The layered article of the present invention has an index A, which is represented by the equation (1), of $0.75 \times 10^{-6}$ $m^3/g$ or less.

$$A=T/G \qquad (1)$$

In the equation (1), T represents the thickness (unit: m) of the fused portion and G represents a basis weight (unit: $g/m^2$). The basis weight means the total basis weight of the entirety of the layered article of the present invention.

The index A is preferably from $0.20 \times 10^{-6}$ $m^3/g$ to $0.72 \times 10^{-6}$ $m^3/g$, more preferably from $0.30 \times 10^{-6}$ $m^3/g$ to $0.70 \times 10^{-6}$ $m^3/g$, particularly preferably from $0.40 \times 10^{-6}$ $m^3/g$ to $0.65 \times 10^{-6}$ $m^3/g$, most preferably from $0.50 \times 10^{-6}$ $m^3/g$ to $0.60 \times 10^{-6}$ $m^3/g$.

When the index A falls within the above-mentioned range, the layered article of the present invention has more sufficient flexibility and can achieve a more satisfactory touch feeling.

The index A is an indicator appropriately reflecting the flexibility of a layered article including at least one layer including a non-woven fabric of fiber, and having a fused portion like the layered article of the present invention. A state in which the index A is smaller reflects a state in which the amount of a material for the layered article of the fused portion in a thickness direction thereof is smaller than the amount of the material for the layered article of a non-fused portion in a thickness direction thereof. Such portion having the smaller amount of the material for the layered article serves as a starting point of bending, and hence the flexibility is improved. In the layered article of the present invention, an index A at a level of $0.75 \times 10^{-6}$ $m^3/g$ or less can be said to reflect a state in which the layered article is particularly excellent in flexibility as a practical effect. Although the index A is desirably as small as possible, a lower limit value for the index A is preferably $0.50 \times 10^{-6}$ $m^3/g$ as described above because when the index is excessively small, a problem in that the layered article ruptures at the time of its bending may occur.

The T representing the thickness (unit: m) of the fused portion is from $5 \times 10^{-6}$ m to $28 \times 10^{-6}$ m, preferably from $10 \times 10^{-6}$ m to $25 \times 10^{-6}$ m, most preferably from $15 \times 10^{-6}$ m to $22 \times 10^{-6}$ m. When the T falls within the above-mentioned range, the layered article of the present invention has more sufficient flexibility and can achieve a more satisfactory touch feeling.

The G representing the basis weight (unit: $g/m^2$) is preferably 50 $g/m^2$ or less, more preferably 48 $g/m^2$ or less, still more preferably from 10 $g/m^2$ to 46 $g/m^2$, particularly preferably from 20 $g/m^2$ to 43 $g/m^2$, most preferably from 30 $g/m^2$ to 40 $g/m^2$. When the G falls within the above-mentioned range, the layered article of the present invention has more sufficient flexibility and can achieve a more satisfactory touch feeling.

One embodiment of the layered article of the present invention is a hook-and-loop fastener female member. The hook-and-loop fastener female member includes an engaging layer engageable with a male member (sometimes referred to as "mechanical hook member") . The engaging layer of the hook-and-loop fastener female member is specifically a layer on which an engaging hook (or something having properties equivalent to those of the engaging hook) of a hook-and-loop fastener male member is engageable. A product including the hook-and-loop fastener female member and a hook-and-loop fastener male member configured to engage with the hook-and-loop fastener female member serves as a hook-and-loop fastener.

The hook-and-loop fastener female member preferably includes the engaging layer engageable with a hook-and-loop fastener male member and a physical property layer configured to hold the engaging layer. The hook-and-loop fastener female member may include any appropriate other member in addition to such engaging layer and physical property layer, as long as the effect of the present invention is not impaired. The hook-and-loop fastener female member is preferably formed of the engaging layer engageable with a hook-and-loop fastener male member and the physical property layer configured to hold the engaging layer.

The thickness of the hook-and-loop fastener female member may be set to any appropriate thickness depending on the purpose. Typically, the thickness of the hook-and-loop fastener female member is preferably from 0.2 mm to 5.0 mm, more preferably from 0.3 mm to 4.0 mm, still more preferably from 0 .5 mm to 3 . 0 mm, particularly preferably from 0.5 mm to 2.0 mm.

The engaging layer includes a non-woven fabric of fiber. The number of layers of the engaging layer may be only one, or may be two or more. The engaging layer is preferably formed only of the non-woven fabric of fiber.

The number of kinds of the non-woven fabric of fiber included in the engaging layer may be only one, or may be two or more.

Examples of the non-woven fabric of fiber included in the engaging layer include a spunbonded non-woven fabric, a thermally bonded non-woven fabric, a bonded and joined non-woven fabric, an air-through non-woven fabric, a meltblown non-woven fabric, a spunbonded meltblown spunbonded non-woven fabric, a spunbonded meltblown meltblown spunbonded non-woven fabric, an unjoined non-woven fabric, an electrospun non-woven fabric, a flashspun non-woven fabric (e.g., TYVEK™ from DuPont), and a carded non-woven fabric. Of the above-mentioned non-woven fabrics, a spunbonded non-woven fabric, a thermally bonded non-woven fabric, a bonded and joined non-woven fabric, an air-through non-woven fabric, a meltblown non-woven fabric, a spunbonded meltblown spunbonded non-woven fabric, or a spunbonded meltblown meltblown spunbonded non-woven fabric is preferred, a spunbonded non-woven fabric or an air-through non-woven fabric is more preferred, and a spunbonded non-woven fabric is still more preferred. When, for example, a thermal point-bonded spunbonded non-woven fabric or an air-through non-woven fabric is used as the non-woven fabric of fiber included in the engaging layer, pieces of the fiber forming the non-woven fabric included in the engaging layer can have mutual bonding points . With this, when the hook-and-loop fastener female member has an embossed pattern, not only pieces of the fiber forming the non-woven fabric included in the engaging layer have firm mutual bonding points in the embossed pattern portions as a result of embossing treatment, but also pieces of the fiber forming the non-woven fabric included in the engaging layer have mutual bonding points in a region free of the embossed pattern. When such structure can be achieved, the hook-and-loop fastener female member has more sufficient flexibility, can achieve a more satisfactory touch feeling, and is excellent in engaging force with a hook-and-loop fastener male member.

In the case where the non-woven fabric of fiber included in the engaging layer is a spunbonded non-woven fabric, the number of bonding points per unit area to be recognized in the region free of the embossed pattern in observation of the non-woven fabric of fiber included in the engaging layer with an optical microscope in a 17 mm×13 mm field of view (at a magnification of 7.5) is preferably from 10 to 200, more preferably from 30 to 150, still more preferably from 50 to 100. In the case where the non-woven fabric of fiber included in the engaging layer is a spunbonded non-woven fabric, when the number of bonding points per unit area to be recognized in observation of the non-woven fabric of fiber included in the engaging layer with an optical microscope falls within the above-mentioned range, the hook-and-loop fastener female member has more sufficient flexibility and can achieve a more satisfactory touch feeling, and fluffing of the engaging layer after peeling after engagement with a hook-and-loop fastener male member can be more effectively suppressed.

In the case where the non-woven fabric of fiber included in the engaging layer is an air-through non-woven fabric, the number of bonding points per unit area to be recognized in the region free of the embossed pattern in observation of the non-woven fabric of fiber included in the engaging layer with an SEM in a 1.3 mmxl.0 mm field of view (at a magnification of 100) is preferably 1 or more, more preferably from 2 to 100, still more preferably from 5 to 50. In the case where the non-woven fabric of fiber included in the engaging layer is an air-through non-woven fabric, when the number of bonding points per unit area to be recognized in observation of the non-woven fabric of fiber included in the engaging layer with an SEM falls within the above-mentioned range, the hook-and-loop fastener female member has more sufficient flexibility and can achieve a more satisfactory touch feeling, and fluffing of the engaging layer after peeling after engagement with a hook-and-loop fastener male member can be more effectively suppressed.

The non-woven fabric of fiber included in the engaging layer may contain fiber that is a homogeneous structural body, or may contain composite fiber that is a bicomponent structural body, such as a core-sheath structure, a side-by-side structure, a sea-island structure, or any other bicomponent structure. Detailed descriptions of the non-woven fabric may be found in, for example, "Nonwoven Fabric Primer and Reference Sampler," E.A. Vaughn, Association of the Nonwoven Fabrics Industry, third edition (1992) .

Any appropriate fiber may be adopted as the fiber forming the non-woven fabric included in the engaging layer as long as the effect of the present invention is not impaired. For example, such fiber contains polyolefin (such as polypropylene or polyethylene), polyester, polyamide, polyurethane, an elastomer, rayon, cellulose, acrylic , a copolymer thereof, or a blend thereof, or a mixture thereof. Such fiber includes preferably at least one kind selected from fiber of polyolefin (polyolefin fiber), fiber of polyester (polyester fiber), and composite fiber of two or more kinds of resins selected from polyolefin and polyester because the effect of the present invention can be more effectively expressed.

Examples of the polyolefin fiber include polypropylene fiber, polyethylene fiber, and $\alpha$-olefin copolymer fiber. The polyolefin fiber is preferably polypropylene fiber or polyethylene fiber, more preferably polypropylene fiber because the effect of the present invention can be more effectively expressed.

Examples of the polyester fiber include polyethylene terephthalate (PET) fiber, polylactic acid fiber, and polyglycolic acid fiber. The polyester fiber is preferably polyethylene terephthalate (PET) fiber because the effect of the present invention can be more effectively expressed.

Examples of the composite fiber of two or more kinds of resins selected from polyolefin and polyester include fiber having a core-sheath structure, fiber having a side-by-side structure, and hollow fiber. As used herein, the term "composite fiber of two or more kinds of resins selected from polyolefin and polyester" means composite fiber of resins that are two or more kinds of polyolefin, composite fiber of resins that are two or more kinds of polyester, or composite fiber of resins that are one or more kinds of polyolefin and one or more kinds of polyester.

Specific examples of the composite fiber of two or more kinds of resins selected from polyolefin and polyester include: fiber having a core-sheath structure in which its core portion contains one of two kinds of polyolefin and its sheath portion contains the other; fiber having a core-sheath structure in which its core portion contains polyester and its sheath portion contains polyolefin; and fiber in which polyolefin and polyester form a side-by-side structure.

The fiber forming the non-woven fabric included in the engaging layer may be crimpable fiber. An example of the crimpable fiber is fiber containing two components having different freezing points, the fiber having a side-by-side structure or an unevenly distributed core-sheath structure, the fiber expressing fine coiled crimps each having a relatively small radius because the component having the higher freezing point first solidifies and shrinks at the time of a phase change from a molten state to a solid state.

The fiber forming the non-woven fabric included in the engaging layer may contain any appropriate other component as long as the effect of the present invention is not impaired. Examples of such other component include other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. Those components may be used alone or in combination thereof. The content of the other component in the fiber forming the non-woven fabric included in the engaging layer is preferably 10 wt% or less, more preferably 7 wt% or less, still more preferably 5 wt% or less, particularly preferably 2 wt% or less, most preferably 1 wt% or less.

In the hook-and-loop fastener female member, the density of the non-woven fabric in the engaging layer is preferably from 5 $kg/m^3$ to 100 $kg/m^3$, more preferably from 10 $kg/m^3$ to 100 $kg/m^3$, still more preferably from 10 $kg/m^3$ to 80 $kg/m^3$, still more preferably from 10 $kg/m^3$ to 70 $kg/m^3$, particularly preferably from 10 $kg/m^3$ to 60 $kg/m^3$, most preferably from 20 $kg/m^3$ to 50 $kg/m^3$, because the effect of the present invention can be more effectively expressed. In the hook-and-loop fastener female member, when the density of the non-woven fabric in the engaging layer falls within the above-mentioned range, the flexibility becomes more sufficient, a more satisfactory touch feeling can be achieved, and the engaging force with a hook-and-loop fastener male member is more excellent, and hence, in a disposable diaper or the like, the problem of slippage, for example, at the time of wearing or after excretion can be effectively eliminated. In the hook-and-loop fastener female member, when the density of the non-woven fabric in the engaging layer is lower than 5 $kg/m^3$, there is a fear that a hook-and-loop fastener male member may be hardly hooked or productivity may be poor, leading to an increased cost. When the density of the non-woven fabric in the engaging layer is higher than 100 $kg/m^3$, a state in which the fiber of the non-woven fabric of the hook-and-loop fastener female member is densely packed is established, and hence there is a fear that it may be difficult to insert the engaging portion of a hook-and-loop fastener male member into the hook-and-loop fastener female member and an excellent engaging force cannot be expressed. The density ($kg/m^3$) of the non-woven fabric in the engaging layer is a value calculated from the basis weight (X $g/m^2$) of the non-woven fabric and the thickness (Y mm) of the non-woven fabric. More specifically, the density ($kg/m^3$) of the non-woven fabric in the engaging layer is calculated as X/Y ($kg/m^3$).

In the hook-and-loop fastener female member, the diameter of the fiber (hereinafter sometimes referred to simply as "fiber diameter") of the non-woven fabric in the engaging layer is preferably from 5 $\mu$m to 60 $\mu$m, more preferably from 10 $\mu$m to 60 $\mu$m, still more preferably from 10 $\mu$m to 50 $\mu$m, still more preferably from 10 $\mu$m to 40 $\mu$m, particularly preferably from 15 $\mu$m to 40 $\mu$m, most preferably from 20 $\mu$m to 40 $\mu$m, because the effect of the present invention can be more effectively expressed. In the hook-and-loop fastener female member, when the diameter of the fiber of the non-woven fabric in the engaging layer falls within the above-mentioned range, the flexibility becomes more sufficient, a more satisfactory touch feeling can be achieved, and the engaging force with a hook-and-loop fastener male member is more excellent, and hence, in a disposable diaper or the like, the problem of slippage, for example, at the time of wearing or after excretion can be effectively eliminated. In the hook-and-loop fastener female member, when the diameter of the fiber of the non-woven fabric in the engaging layer is smaller than 5 $\mu$m, there is a fear that the engaging force with a hook-and-loop fastener male member may lower. When the diameter of the fiber of the non-woven fabric in the engaging layer is larger than 60 $\mu$m, there is a fear that engagement with a hook-and-loop fastener male member may become difficult or production speed may lower, leading to an increased cost.

In the hook-and-loop fastener female member, the basis weight of the non-woven fabric in the engaging layer is preferably from 10 $g/m^2$ to 60 $g/m^2$, more preferably from 12 $g/m^2$ to 50 $g/m^2$, still more preferably from 15 $g/m^2$ to 40 $g/m^2$, particularly preferably from 15 $g/m^2$ to 30 $g/m^2$, most preferably from 15 $g/m^2$ to 25 $g/m^2$. In the hook-and-loop fastener female member, when the basis weight of the non-woven fabric in the engaging layer falls within the above-mentioned range, there can be provided a hook-and-loop fastener female member that has more sufficient flexibility, can achieve a more satisfactory touch feeling, and is more excellent in engaging force with a hook-and-loop fastener male member.

Any appropriate material may be adopted as a material for the physical property layer as long as the effect of the present invention is not impaired. Examples of the material for the physical property layer include a non-woven fabric of fiber and a film, which are preferred because the effect of the present invention can be more expressed. Of those, a non-woven fabric of fiber is more preferred because the effect of the present invention can be more effectively expressed. That is, the hook-and-loop fastener female member is preferably formed only of non-woven fabrics because the effect of the present invention can be more effectively expressed.

When the material for the physical property layer is a non-woven fabric of fiber, the number of kinds of the non-woven fabric may be only one, or may be two or more.

When the material for the physical property layer is a non-woven fabric of fiber, examples of the non-woven fabric include a spunbonded non-woven fabric, a thermally bonded non-woven fabric, a bonded and joined non-woven fabric, an air-through non-woven fabric, a meltblown non-woven fabric, a spunlace non-woven fabric, a spunbonded meltblown spunbonded non-woven fabric, a spunbonded meltblown meltblown spunbonded non-woven fabric, an unjoined non-woven fabric, an electrospun non-woven fabric, a flashspun non-woven fabric (such as TYVEK™ from Du-Pont), and a carded non-woven fabric.

When the material for the physical property layer is a non-woven fabric of fiber, the non-woven fabric may contain fiber that is a homogeneous structural body, or may contain composite fiber that is a bicomponent structural body, such as a core-sheath structure, a side-by-side structure, a sea-island structure, or any other bicomponent structure. Detailed descriptions of the non-woven fabric may be found in, for example, "Nonwoven Fabric Primer and Reference Sampler, " E.A. Vaughn, Association of the Nonwoven Fabrics Industry, third edition (1992).

When the material for the physical property layer is a non-woven fabric of fiber, any appropriate fiber may be adopted as the fiber as long as the effect of the present invention is not impaired. For example, such fiber contains polyolefin (such as polypropylene or polyethylene), polyester, polyamide, polyurethane, an elastomer, rayon, cellulose, acrylic, a copolymer thereof, or a blend thereof, or a mixture thereof. Such fiber includes preferably at least one kind selected from fiber of polyolefin (polyolefin fiber), fiber of polyester (polyester fiber), and composite fiber of two or more kinds of resins selected from polyolefin and polyester because the effect of the present invention can be more effectively expressed.

Examples of the polyolefin fiber include polypropylene fiber, polyethylene fiber, and $\alpha$-olefin copolymer fiber. The polyolefin fiber is preferably polypropylene fiber or polyethylene fiber, more preferably polypropylene fiber because the effect of the present invention can be more effectively expressed.

Examples of the polyester fiber include polyethylene terephthalate (PET) fiber, polylactic acid fiber, and polyglycolic acid fiber. The polyester fiber is preferably polyethylene terephthalate (PET) fiber because the effect of the present invention can be more effectively expressed.

Examples of the composite fiber of two or more kinds of resins selected from polyolefin and polyester include fiber having a core-sheath structure, fiber having a side-by-side structure, and hollow fiber. As used herein, the term "composite fiber of two or more kinds of resins selected from polyolefin and polyester" means composite fiber of resins that are two or more kinds of polyolefin, composite fiber of resins that are two or more kinds of polyester, or composite fiber of resins that are one or more kinds of polyolefin and one or more kinds of polyester.

Specific examples of the composite fiber of two or more kinds of resins selected from polyolefin and polyester include: fiber having a core-sheath structure in which its core portion contains one of two kinds of polyolefin and its sheath portion contains the other; fiber having a core-sheath structure in which its core portion contains polyester and its sheath portion contains polyolefin; and fiber in which polyolefin and polyester form a side-by-side structure.

When the material for the physical property layer is a non-woven fabric of fiber, the fiber forming the non-woven fabric may be crimpable fiber. An example of the crimpable fiber is fiber containing two components having different freezing points, the fiber having a side-by-side structure or an unevenly distributed core-sheath structure, the fiber expressing fine coiled crimps each having a relatively small radius because the component having the higher freezing point first solidifies and shrinks at the time of a phase change from a molten state to a solid state.

When the material for the physical property layer is a non-woven fabric of fiber, the fiber forming the non-woven fabric may contain any appropriate other component as long as the effect of the present invention is not impaired. Examples of such other component include other polymers, a tackifier, a plasticizer, an antidegradant, a pigment, a dye, an antioxidant, an antistatic agent, a lubricant, a blowing agent, a heat stabilizer, a light stabilizer, an inorganic filler, and an organic filler. Those components may be used alone or in combination thereof. The content of the other component in the fiber forming the non-woven fabric included in the engaging layer is preferably 10 wt% or less, more preferably 7 wt% or less, still more preferably 5 wt% or less, particularly preferably 2 wt% or less, most preferably 1 wt% or less.

When the material for the physical property layer is a film, any appropriate material may be adopted as a material for the film as long as the effect of the present invention is not impaired. Examples of such material include an unstretched polypropylene film, a stretched polypropylene film, and a polyethylene film each having a thickness of from 10 $\mu$m to 60 $\mu$m, which are preferred because the effect of the present invention can be more effectively expressed.

When the physical property layer is a non-woven fabric of fiber, the non-woven fabric in the physical property layer has a basis weight of preferably from 10 g/m$^2$ to 40 g/m$^2$, more preferably from 10 g/m$^2$ to 30 g/m$^2$, still more preferably from 10 g/m$^2$ to 25 g/m$^2$, particularly preferably from 10 g/m$^2$ to 20 g/m$^2$. In the case where the physical property layer is a non-woven fabric of fiber, when the basis weight of the non-woven fabric in the physical property layer falls within the above-mentioned range, the flexibility becomes more sufficient, a more satisfactory touch feeling can be achieved, shrinkage deformation in a width direction hardly occurs during web handling, cost competitiveness is excellent, printability is satisfactory, the see-through property of printing is satisfactory, pressure-sensitive adhesive application is easy, and the applied pressure-sensitive adhesive hardly exudes to the engaging surface.

When the physical property layer is a non-woven fabric of fiber, the diameter of the fiber is preferably 40 $\mu$m or less, more preferably from 1 $\mu$m to 40 $\mu$m, still more preferably from 1 $\mu$m to 30 $\mu$m, particularly preferably from 1 $\mu$m to 25 $\mu$m, most preferably from 1 $\mu$m to 20 $\mu$m. In the case where the physical property layer is a non-woven fabric of fiber, when the diameter of the fiber falls within the above-mentioned range, the flexibility becomes more sufficient, a more satisfactory touch feeling can be achieved, shrinkage deformation in a width direction hardly occurs during web handling, cost competitiveness is excellent, printability is satisfactory, the see-through property of printing is satisfactory, pressure-sensitive adhesive application is easy, and the applied pressure-sensitive adhesive hardly exudes to the engaging surface. In the case where the physical property layer is a non-woven fabric of fiber, when the diameter of the fiber is larger than 40 $\mu$m, there is a fear that shrinkage deformation in a width direction may be liable to occur during web handling, cost competitiveness may be inferior, printability may be poor, pressure-sensitive adhesive application may be difficult, and the applied pressure-sensitive adhesive may have a risk of exuding to the engaging surface.

When the physical property layer is a non-woven fabric of fiber, in the hook-and-loop fastener female member, the density of the non-woven fabric is preferably from 5 kg/m$^3$ to 200 kg/m$^3$, more preferably from 20 kg/m$^3$ to 150 kg/m$^3$, still more preferably from 50 kg/m$^3$ to 150 kg/m$^3$, still more preferably from 50 kg/m$^3$ to 120 kg/m$^3$, particularly preferably from 60 kg/m$^3$ to 120 kg/m$^3$, most preferably from 70 kg/m$^3$ to 120 kg/m$^3$, because the effect of the present invention can be more effectively expressed. The density (kg/m$^3$) of the non-woven fabric in the physical property layer is a value calculated from the basis weight (X g/m$^2$) of the non-woven fabric and the thickness (Y mm) of the non-woven fabric. More specifically, the density (kg/m$^3$) of the non-woven fabric in the physical property layer is calculated as X/Y (kg/m$^3$).

When the physical property layer is a non-woven fabric of fiber, a laminate of different kinds of non-woven fabrics of fiber (e.g., a laminate of spunbonded non-woven fabric/meltblown non-woven fabric/spunbonded non-woven fabric) may be adopted.

When the diameter of the fiber significantly varied in a thickness direction (e.g., SMS or SSMMS), the same number of diameters of the fiber were measured for N=5 or more in each piece of the fiber, and the average value of the measured diameters was defined as the diameter of the fiber. A portion having a locally small thickness due to heat fusion or the like as in spunbond, spunmelt, or the like was not included.

When the physical property layer is a film, its thickness is preferably 60 $\mu$m or less, more preferably from 10 $\mu$m to 50 $\mu$m, still more preferably from 10 $\mu$m to 40 $\mu$m, particularly preferably from 10 $\mu$m to 30 $\mu$m, most preferably from 15 $\mu$m to 25 $\mu$m. In the case where the physical property layer is a film, when its thickness falls within the above-mentioned range, the flexibility becomes more sufficient, a more satisfactory touch feeling can be achieved, shrinkage deformation in a width direction hardly occurs during web handling, cost competitiveness is excellent, the see-through property of printing is satisfactory, and pressure-sensitive adhesive application is easy. In the case where the physical property layer is a film, when its thickness is larger than 60 $\mu$m, there is a fear that cost competitiveness maybe inferior and the see-through property of printing may degrade.

In the hook-and-loop fastener female member, the total basis weight, which is the sum of the basis weight of the non-woven fabric in the engaging layer and the basis weight of the non-woven fabric in the physical property layer, is preferably 60 g/m$^2$ or less, more preferably from 10 g/m$^2$ to 57 g/m$^2$, still more preferably from 15 g/m$^2$ to 53 g/m$^2$, particularly preferably from 20 g/m$^2$ to 50 g/m$^2$, most preferably from 30 g/m$^2$ to 47 g/m$^2$. In the hook-and-loop fastener female member, when the total basis weight, which is the sum of the basis weight of the non-woven fabric in the engaging layer and the basis weight of the non-woven fabric in the physical property layer, falls within the above-mentioned range, the flexibility becomes more sufficient, a more satisfactory touch feeling can be achieved, and the engaging force with a hook-and-loop fastener male member is more excellent. In addition, in the hook-and-loop fastener female member, when the total basis weight, which is the sum of the basis weight of the non-woven fabric in the engaging layer and the basis weight of the non-woven fabric in the physical property layer, falls within the above-mentioned range, the flexibility becomes more sufficient, a more satisfactory touch feeling can be achieved, shrinkage deformation in a width direction is still less liable to occur during web handling, cost competitiveness is more excellent, printability is more satisfactory, the see-through property of printing is more satisfactory, pressure-sensitive adhesive application is easier, and the applied pressure-sensitive adhesive is still less liable to exude to the engaging surface.

The density of the hook-and-loop fastener female member is preferably 110 kg/m$^3$ or less, more preferably from 5 kg/m$^3$ to 110 kg/m$^3$, still more preferably from 10 kg/m$^3$ to 110 kg/m$^3$, still more preferably from 10 kg/m$^3$ to 80 kg/m$^3$, still more preferably from 10 kg/m$^3$ to 70 kg/m$^3$, particularly preferably from 10 kg/m$^3$ to 60 kg/m$^3$, most preferably from 20 kg/m$^3$ to 50 kg/m$^3$. When the density of the hook-and-loop fastener female member falls within the above-mentioned range, the flexibility becomes more sufficient, a more satisfactory touch feeling can be achieved, and the engaging force with a hook-and-loop fastener male member is more excellent, and hence, in a disposable diaper or the like, the problem of slippage, for example, at the time of wearing or after excretion can be effectively eliminated. When the density of the hook-and-loop fastener female member is more than 110 kg/m$^3$, a state in which the fiber of the non-woven fabric of the hook-and-loop fastener female member is densely packed is established, and hence there is a fear that it may be difficult to insert the engaging portion of a hook-and-loop fastener male member into the hook-and-loop fastener female member and an excellent engaging force cannot be expressed. The density ($kg/m^3$) of the hook-and-loop fastener female member is a value calculated from the basis weight ($X$ $g/m^2$) of the non-woven fabric in the hook-and-loop fastener female member and the thickness ($Y$ mm) of the non-woven fabric in the hook-and-loop fastener female member. More specifically, the density ($kg/m^3$) of the hook-and-loop fastener female member is calculated as $X/Y$ ($kg/m^3$).

In the hook-and-loop fastener female member, it is preferred that the surface of the fiber forming the non-woven fabric included in the engaging layer and the surface of the physical property layer on the engaging layer side contain the same kind of polymer. When the surface of the fiber forming the non-woven fabric included in the engaging layer and the surface of the physical property layer on the engaging layer side contain the same kind of polymer, there can be provided a hook-and-loop fastener female member that has more sufficient flexibility, can achieve a more satisfactory touch feeling, and is more excellent in engaging force with a hook-and-loop fastener male member. In this case, the "surface of the fiber forming the non-woven fabric included in the engaging layer" may be any surface of the fiber, and encompasses, for example, a sheath portion in fiber having a core-sheath structure.

When the surface of the fiber forming the non-woven fabric included in the engaging layer and the surface of the physical property layer on the engaging layer side contain the same kind of polymer, any appropriate polymer may be adopted as the polymer as long as the effect of the present invention is not impaired. Such polymer is preferably polyolefin. When the surface of the fiber forming the non-woven fabric included in the engaging layer and the surface of the physical property layer on the engaging layer side contain the same kind of polyolefin, there can be provided a hook-and-loop fastener female member that has more sufficient flexibility, can achieve a more satisfactory touch feeling, and is more excellent in engaging force with a hook-and-loop fastener male member.

In the hook-and-loop fastener female member, when, for example, a spunbonded non-woven fabric or an air-through non-woven fabric is used as the non-woven fabric of fiber included in the engaging layer, pieces of the fiber forming the non-woven fabric included in the engaging layer can have mutual bonding points. With this, when the hook-and-loop fastener female member has an embossed pattern, not only pieces of the fiber forming the non-woven fabric included in the engaging layer have firm mutual bonding points in the embossed pattern portions (the portions of the plurality of embossments 10 in FIG. 1) as a result of embossing treatment, but also pieces of the fiber forming the non-woven fabric included in the engaging layer have mutual bonding points in the region free of the embossed pattern (the region 20 free of the embossed pattern in FIG. 1). When such structure can be achieved, the hook-and-loop fastener female member has more sufficient flexibility, can achieve a more satisfactory touch feeling, and is more excellent in engaging force with a hook-and-loop fastener male member.

<<2. Application of Layered Article of the Present Invention>>

The layered article of the present invention can provide a hook-and-loop fastener by being combined with a hook-and-loop fastener male member configured to engage with the layered article. That is, a hook-and-loop fastener of the present invention includes the layered article of the present invention and a hook-and-loop fastener male member configured to engage with the layered article. In addition, the layered article of the present invention can be used for any appropriate article in which the effect of the present invention can be effectively utilized. A typical example of such article is a sanitary article. That is, the sanitary article includes the layered article of the present invention. Examples of such sanitary article include a diaper (in particular, a disposable diaper), a supporter, and a mask.

<<3. Production Method for LayeredArticle of the Present Invention>>

The layered article of the present invention may be produced by any appropriate method as long as the effect of the present invention is not impaired. One preferred method of producing the layered article of the present invention is, for example, a method involving fusing at least part of a single-layer body or laminate including at least one layer including a non-woven fabric of fiber through ultrasonic welding. In the case where at least part of the single-layer body or the laminate including at least one layer including the non-woven fabric of fiber is fused through the ultrasonic welding, the production speed of the layered article can be increased as compared to, for example, that in the case where at least part of the single-layer body or the laminate is fused through heat fusion. In addition, when at least part of the single-layer body or the laminate including at least one layer including the non-woven fabric of fiber is fused through the ultrasonic welding, the layered article to be obtained has more sufficient flexibility and can achieve a more satisfactory touch feeling.

Any appropriate ultrasonic welding may be adopted as the ultrasonic welding as long as the effect of the present invention is not impaired.

In the ultrasonic welding, members to be bonded are arranged between a part generally referred to as "horn", the part being configured to feed vibration energy with an ultrasonic wave, and a roll-shaped part generally referred to as "anvil" or "rotating anvil". In many cases, the horn is arranged vertically above the members to be bonded and the rotating anvil. The horn typically vibrates at from 20,000 Hz to 40,000 Hz to transfer energy typically in the form of frictional heat to the members to be bonded under pressure . Part of at least one of the members to be bonded is softened or melted by the frictional heat and the pressure, and hence the materials are bonded to each other.

A pressing force between the horn and the rotating anvil in the ultrasonic welding is preferably from 100 N to 1,500 N, more preferably from 300 N to 1,300 N, still more preferably from 500 N to 1,100 N, particularly preferably from 700 N to 1,000 N. When the pressing force between the horn and the rotating anvil in the ultrasonic welding falls within the above-mentioned range, the layered article to be obtained has more sufficient flexibility and can achieve a more satisfactory touch feeling. In addition, the production speed of the layered article of the present invention can be further increased.

One preferred kind of ultrasonic welding is generally known as "continuous ultrasonic welding." The continuous ultrasonic welding is typically used for sealing members to be bonded that can be supplied into a bonding apparatus in a substantially continuous manner. In the continuous ultrasonic welding, the horn is typically fixed and the members to be bonded move directly below the horn. In one kind of continuous ultrasonic welding, the fixed horn and a rotating anvil surface are used. During the continuous ultrasonic welding, the members to be bonded are pulled between the horn and the rotating anvil. The horn typically extends in its lengthwise direction toward the members to be bonded, and its vibration moves along the horn in its axial direction to the materials.

In another preferred kind of ultrasonic welding bonding, the horn is a rotation type, has a cylindrical shape, and rotates about its lengthwise direction axis. Input vibration is present in the axial direction of the horn and output vibration is present in the radial direction of the horn. The horn is arranged so as to be close to the rotating anvil, and the anvil can also typically rotate so that the members to be bonded may pass a space between cylindrical surfaces at a line velocity substantially equal to the tangential velocity of the cylindrical surfaces.

The ultrasonic welding is described in, for example, JP 2008-526552 A, JP 2010-195044 A, JP 2013-231249 A, JP 2015-16294 A, and US 5976316 A, and the contents of the disclosures are incorporated herein by reference.

One preferred method of producing the layered article of the present invention is a method involving fusing at least part of the single-layer body or the laminate including at least one layer including the non-woven fabric of fiber through the ultrasonic welding, and the ultrasonic welding is more preferably performed with a heated rotating anvil. When the ultrasonic welding is performed with a rotating anvil that has not been heated, in the case where the fusion is continuously performed, heat accumulates in each of the horn and the rotating anvil with time. Accordingly, a fusion strength varies in each of the flow direction and width direction of the layered article, and hence a variation in quality of the product occurs. When the ultrasonic welding is performed with the heated rotating anvil, the heat accumulation in each of the horn and the rotating anvil with time can be reduced. Accordingly, the variation in fusion strength can be suppressed, and hence the variation in quality of the product can be reduced. In addition, when the heated rotating anvil is used, the heat of the rotating anvil can be utilized as energy needed for the fusion, and hence more energy can be used for the fusion. As a result, the production speed can be increased.

When the ultrasonic welding is performed with the heated rotating anvil, its heating temperature is preferably from 30°C to 150°C, more preferably from 35°C to 120°C, still more preferably from 40°C to 100°C, particularly preferably from 50°C to 90°C, most preferably from 60°C to 80°C. When the heating temperature of the rotating anvil falls within the above-mentioned range, the heat accumulation in each of the horn and the rotating anvil with time can be reduced. Accordingly, the variation in fusion strength can be suppressed, and hence the variation in quality of the product can be reduced. In addition, the heat of the rotating anvil can be utilized as energy needed for the fusion, and hence more energy can be used for the fusion. As a result, the production speed can be increased. In addition, when the heating temperature of the rotating anvil falls within the above-mentioned range, the layered article to be obtained has more sufficient flexibility and can achieve a more satisfactory touch feeling.

When the ultrasonic welding is performed with the heated rotating anvil, further, a fluctuation in temperature of the rotating anvil is preferably within ±20°C, more preferably within ±10°C, still more preferably within ±8°C, particularly preferably within ±5°C, most preferably within ±2°C. When the fluctuation in temperature of the rotating anvil falls within the above-mentioned range, the variation in fusion strength can be more suppressed, and hence the variation in quality of the product can be more reduced. Further, when the fluctuation in temperature of the rotating anvil falls within the above-mentioned range, the layered article to be obtained has more sufficient flexibility and can achieve a more satisfactory touch feeling.

Any appropriate method may be adopted as a method of heating the rotating anvil as long as the effect of the present invention is not impaired. Examples of such method include an induction heat generation system, an electric heating system, a heat medium circulation system, and a steam heating system.

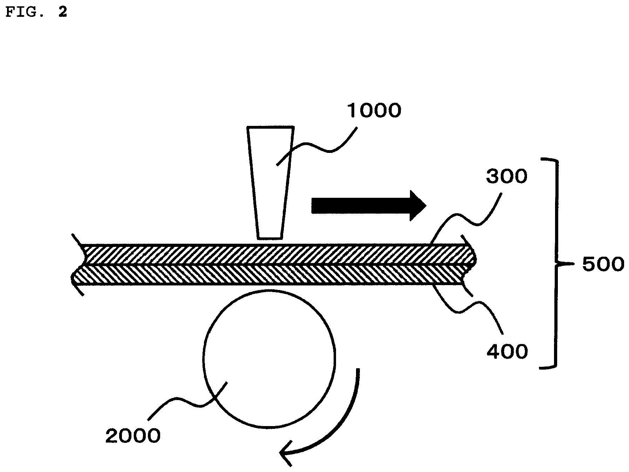

FIG. 2 is a schematic sectional view for illustrating an example of a preferred method of producing the layered article of the present invention. In FIG. 2, reference numeral 1000 represents a horn and reference numeral 2000 represents a rotating anvil. A laminate 500 of an engaging layer 300 and a physical property layer 400 flows between the horn 1000 and the rotating anvil 2000 in a direction indicated by the arrow, and ultrasonic welding is performed between the horn **1000** and the rotating anvil **2000.** The horn **1000** and the engaging layer **300** may be in contact with each other, or may be out of contact with each other. The rotating anvil **2000** and the physical property layer **400** may be in contact with each other, or may be out of contact with each other. The laminate **500** that has passed a space between the horn **1000** and the rotating anvil **2000** can be the layered article of the present invention (e.g., a hook-and-loop fastener female member).

In the method involving fusing at least part of the single-layer body or the laminate including at least one layer including the non-woven fabric of fiber through the ultrasonic welding, the ratio of the area of a fused portion obtained by the fusion through the ultrasonic welding to the area of the entire surface of the layered article to be produced (hereinafter sometimes referred to as "fused area ratio") is preferably 50% or less, more preferably from 1% to 40%, still more preferably from 5% to 35%, particularly preferably from 10% to 30%, most preferably from 15% to 25%. When the above-mentioned fused area ratio falls within the above-mentioned range, the layered article of the present invention has more sufficient flexibility and can achieve a more satisfactory touch feeling. In addition, when the above-mentioned fused area ratio falls within the above-mentioned range, the production speed can be increased. As described later, when an embossed pattern is provided on the surface of the rotating anvil, the "fused area ratio" is sometimes rephrased as an "embossing-fused area ratio."

The surface of the rotating anvil preferably has an embossed pattern. Specific examples of such embossed pattern include a continuous grid shape, a discontinuous grid shape, a continuous curve shape, a discontinuous curve shape, a continuous zigzag shape, a discontinuous zigzag shape, a continuous linear shape, a discontinuous linear shape, a circle shape, an ellipse shape, a hollow circle shape, a hollow ellipse shape, an arc shape, and a hollow arc shape.

The embossed pattern is preferably a discontinuous embossed pattern, more preferably an embossed pattern of an arc shape, because the effect of the present invention can be more effectively expressed. An example of the embossed pattern of an arc shape that the surface of the rotating anvil may have is an embossed pattern that may impart the embossed pattern of an arc shape illustrated in FIG. 1 described in the foregoing to the layered article to be produced.

That is, FIG. **1** is a schematic plan view for illustrating an example of the embossed pattern that may be imparted to the surface of the layered article of the present invention, and the embossed pattern has the plurality of embossments **10** serving as concave portions. An embossed pattern having embossments serving as convex portions that may form the embossments **10** may be provided on the surface of the rotating anvil. The individual embossments serving as convex portions are embossments having no "corners".

The embossment width of each of the plurality of embossments serving as convex portions forming the embossed pattern is preferably from 0.1 mm to 3.0 mm, more preferably from 0.3 mm to 2.0 mm, still more preferably from 0.3 mm to 1.5 mm, particularly preferably from 0.5 mm to 1.5 mm, most preferably from 0.5 mm to 1.0 mm. When the embossment width falls within the above-mentioned range, the layered article of the present invention has more sufficient flexibility and can achieve a more satisfactory touch feeling. The embossment width refers to, for example, a width W of each of the embossments 10 in an MD direction as illustrated in FIG. 1.

The distance between two adjacent embossments in the plurality of embossments forming the embossed pattern on any line in the MD direction is preferably 10 mm or less, more preferably from 1 mm to 10 mm, still more preferably from 1.5 mm to 9 mm, particularly preferably from 2 mm to 8 mm, most preferably from 2.5 mm to 7 mm. When the distance between two adjacent embossments in the plurality of embossments forming the embossed pattern on any line in the MD direction falls within the above-mentioned range, the layered article of the present invention has more sufficient flexibility and can achieve a more satisfactory touch feeling. The distance between two adjacent embossments in the plurality of embossments forming the embossed pattern on a line in the MD direction corresponds to, for example, a distance L between two adjacent embossments on a line P in the MD direction illustrated in FIG. 1 (which may be a line in the MD direction at any position in a CD direction).

The height of each of the embossments serving as convex portions is preferably from 0.1 mm to 2.0 mm, more preferably from 0.2 mm to 1.8 mm, still more preferably from 0.3 mm to 1.5 mm, particularly preferably from 0.5 mm to 1.5 mm, most preferably from 0.7 mm to 1.2 mm. When the height of each of the embossments serving as convex portions falls within the above-mentioned range, the layered article of the present invention has more sufficient flexibility and can achieve a more satisfactory touch feeling.

Examples

The present invention is hereinafter specifically described by way of Examples. However, the present invention is by no means limited to these Examples. In Examples and the like, test and evaluation methods are as described below. In addition, "part (s) " means "part (s) by weight" and "%" means "wt%" unless otherwise stated.

<Measurement of Fiber Diameter>

Through the use of a digital microscope "VHX-1000 " manufactured by Keyence Corporation, a non-woven fabric surface was photographed at a magnification of 500, and fiber diameters were measured for N=5 or more with the image analysis software of the digital microscope . The average value of the measured fiber diameters was defined as a fiber diameter.

<Thickness of Fused Portion formed by Embossed Pattern>

A cross-section exposed by cutting an obtained layered article with a freezing microtome was subjected to conductive treatment. The cross-section was photographed with an FE-SEM (manufactured by Hitachi, Ltd., S-4800, secondary electron image, acceleration voltage: 1 kV), and the thickness of a fused portion was measured. The measurement was performed at measurement points whose number N was equal to 3, and the average of the measured values was defined as the thickness of the fused portion.

<Flexibility>

An obtained layered article was cut into a size measuring 2.5 cm (MD direction) by 50 cm (TD direction) . The cut layered article was formed into a ring shape with its physical property layer surface side being a roll inner side and its TD being a circumference, and end portions thereof were fixed to each other at two places through use of a stapler in such a manner that the end portions did not overlap each other.
A compressive load was applied to the resultant ring body of the layered article from its width direction by sandwiching the ring body between a forefinger and a thumb, and a repulsive force when the ring body was deformed until its diameter became one half of the initial diameter was identified.
○: Low repulsive force and high flexibility.
×: High repulsive force and low flexibility.

[Examples 2 to 6 and Reference Example 1]

Raw non-woven fabrics shown in Table 1 were laminated, and were subjected to embossing treatment with an embossed pattern roll at 23°C and an ultrasonic continuous fusion apparatus manufactured by Herrmann Ultrasonics (frequency: 20 kHz, pressing force: 900 N, speed: 50 m/min) to provide layered articles.
The embossed pattern used was the embossed pattern of an arc shape illustrated in FIG. 1, and the embossment width was 0.8 mm, the maximum value of the distance between two adjacent embossments on a line in the MD direction (maximum embossment-to-embossment distance) was 3.2 mm, the embossing-fused area ratio was 23%, and the thickness of a fused portion formed by the embossed pattern was as shown in Table 1.
The results are shown in Table 1.

[Example 7]

Layered article production was performed in the same manner as in Reference Example 1 except that in Reference Example 1, the temperature of the embossed pattern roll was set to 50°C and the speed was set to 100 m/min.
The results are shown in Table 1.

[Example 8]

Layered article production was performed in the same manner as in Example 6 except that in Example 6, the temperature of the embossed pattern roll was set to 70°C, the pressing force was set to 850 N, and the speed was set to 100 m/min.
The results are shown in Table 1.

[Examples 9 to 11]

Layered article production was performed in the same manner as in Example 8 except that in Example 8, the raw non-woven fabrics were changed as shown in Table 1.
The results are shown in Table 1.

[Example 12]

Layered article production was performed in the same manner as in Example 6 except that in Example 6, the temperature of the embossed pattern roll was set to 80°C, the pressing force was set to 830 N, and the speed was set to 100 m/min.

The results are shown in Table 1.

[Comparative Examples 1 to 3]

Raw non-woven fabrics shown in Table 1 were laminated, and were subjected to embossing treatment with an embossed pattern roll at 200°C and a rubber roll at room temperature (pressing force: 5,000 N, speed: 10 m/min) to provide layered articles.

The embossed pattern used was the embossed pattern of an arc shape illustrated in FIG. 1, and the embossment width was 0.8 mm, the maximum value of the distance between two adjacent embossments on a line in the MD direction (maximum embossment-to-embossment distance) was 3.2 mm, the embossing-fused area ratio was 23%, and the thickness of a fused portion formed by the embossed pattern was as shown in Table 1.

The results are shown in Table 1.

Table 1

| | | Example 1* | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|
| Engaging layer | Fiber composition | PE/PET | PE/PET | PE/PP | PE/PP | PE/PP | PP | PE/PET | PE/PET | PE/PP | PP |
| | Fiber diameter [μm] | 31.6 | 31.6 | 18.2 | 20.1 | 20.1 | 21.8 | 31.6 | 31.6 | 31.0 | 20.0 |
| | Basis weight [g/m²] | 30 | 30 | 22 | 18 | 18 | 18 | 30 | 30 | 30 | 22 |
| Physical property layer | Fiber composition | PE/PP | PE/PP | PE/PP | PE/PP | PE/PP | PP | PE/PP | | | |
| | Fiber diameter [μm] | 19.8 | 19.8 | 18.2 | 19.8 | 18.2 | 19.6 | 19.8 | | | |
| | Basis weight [g/m²] | 20 | 15 | 18 | 15 | 20 | 15 | 15 | | | |
| Total basis weight [g/m²] | | 50 | 45 | 40 | 38 | 38 | 33 | 45 | | | |
| Thickness of fused portion formed by embossed pattern [μm] | | 30.3 | 26.7 | 28.3 | 20.3 | 21.0 | 18.3 | 27.3 | | | |
| A [m³/g] | | $0.61\times10^{-6}$ | $0.59\times10^{-6}$ | $0.71\times10^{-6}$ | $0.54\times10^{-6}$ | $0.55\times10^{-6}$ | $0.56\times10^{-6}$ | $0.61\times10^{-6}$ | | | |
| Number of holes | | 0 | 2 | 3 | 3 | 2 | 4 | 0 | | | |
| Flexibility | | ○ | ○ | ○ | ○ | ○ | ○ | ○ | | | |

| | | Example 8 | Example 9 | Example 10 | Example 11 | Example 12 |
|---|---|---|---|---|---|---|
| Engaging layer | Fiber composition | PP | PP | PP | PP | PP |
| | Fiber diameter [μm] | 21.8 | 14.5 | 30.8 | 31.2 | 21.8 |
| | Basis weight [g/m²] | 18 | 24 | 18 | 24 | 18 |

(continued)

|  |  | Example 8 | Example 9 | Example 10 | Example 11 | Example 12 | Comparative Example 1 | Comparative Example 2 | Comparative Example 3 |
|---|---|---|---|---|---|---|---|---|---|
| Physical property layer | Fiber composition | PP | PP | PP | PP | PP | PE/PP | PE/PP | PP |
|  | Fiber diameter [$\mu$m] | 19.6 | 19.6 | 19.6 | 19.6 | 19.6 | 19.8 | 20.2 | 9 |
|  | Basis weight [g/m$^2$] | 15 | 15 | 15 | 15 | 15 | 15 | 20 | 15 |
| Total basis weight [g/m$^2$] | | 33 | 39 | 33 | 39 | 33 | 45 | 50 | 37 |
| Thickness of fused portion formed by embossed pattern [$\mu$m] | | 18.8 | 25.4 | 20.1 | 24.5 | 18.5 | 58.3 | 50.3 | 35.3 |
| A [m$^3$/g] | | $0.57 \times 10^{-6}$ | $0.65 \times 10^{-6}$ | $0.61 \times 10^{-6}$ | $0.62 \times 10^{-6}$ | $0.56 \times 10^{-6}$ | $1.30 \times 10^{-6}$ | $1.01 \times 10^{-6}$ | $0.95 \times 10^{-6}$ |
| Number of holes | | 0 | 0 | 0 | 0 | 0 | 0 | 0 | 0 |
| Flexibility | | ○ | ○ | ○ | ○ | ○ | × | × | × |
| * Example 1 is not in accordance with the invention | | | | | | | | | |

The layered article of the present invention can be used for any appropriate article in which the effect of the present invention can be effectively utilized. A typical example of such article is a sanitary article. Examples of such sanitary article include a diaper (in particular, a disposable diaper), a supporter, and a mask.

Reference Signs List

| | |
|---|---|
| **10** | embossment |
| **20** | region free of embossed pattern |
| **100** | fused portion |
| **300** | engaging layer |
| **400** | physical property layer |
| **500** | laminate |
| **1000** | horn |
| **2000** | rotating anvil |

**Claims**

1. A layered article, comprising at least one layer including a non-woven fabric of fiber, wherein:

   the layered article has a fused portion (100); and
   the layered article has an index A, which is represented by the equation (1), of $0.75 \times 10^{-6}$ m$^3$/g or less:

   $$A = T/G \qquad (1)$$

   where T represents a thickness (unit: m) of the fused portion and G represents a basis weight (unit: g/m$^2$), **characterized in that** T is from $5 \times 10^{-6}$ m to $28 \times 10^{-6}$ m.

2. The layered article according to claim 1, wherein the index A is from $0.20 \times 10^{-6}$ m$^3$/g to $0.72 \times 10^{-6}$ m$^3$/g.

3. The layered article according to claim 1, wherein the G is 50 g/m$^2$ or less.

4. The layered article according to claim 3, wherein the G is 48 g/m$^2$ or less.

5. The layered article according to claim 1, wherein the fused portion (100) includes a fused portion of pieces of the fiber.

6. The layered article according to claim 1, wherein a number of holes each having a diameter of 100 $\mu$m or more present in the fused portion is 10 holes/m$^2$ or less.

7. The layered article according to claim 1, wherein the fused portion (100) has an embossed pattern.

8. The layered article according to claim 1, wherein the layered article comprises a hook-and-loop fastener female member.

9. The layered article according to claim 8, wherein the layered article comprises:

   an engaging layer (300) engageable with a hook-and-loop fastener male member; and
   a physical property layer (400) configured to hold the engaging layer.

10. The layered article according to claim 9, wherein a sum of a basis weight of a non-woven fabric in the engaging layer and a basis weight of a non-woven fabric in the physical property layer is 60 g/m$^2$ or less.

11. The layered article according to claim 9, wherein a surface of fiber forming a non-woven fabric included in the engaging layer and a surface of the physical property layer on the engaging layer side contain the same kind of polymer.

**12.** A hook-and-loop fastener, comprising:

the layered article of claim 8; and
a hook-and-loop fastener male member configured to engage with the layered article.

**Patentansprüche**

**1.** Mehrlagiger Gegenstand, umfassend mindestens eine Schicht, die einen Vliesstoff aus einer Faser enthält, wobei:

der mehrlagige Gegenstand einen verschmolzenen Teilbereich (100) aufweist; und
der mehrlagige Gegenstand einen Index A, der durch die Gleichung (1) dargestellt ist, von $0{,}75 \times 10^{-6}$ m³/g oder weniger aufweist:

$$A = T/G \qquad (1)$$

wobei T eine Dicke (Einheit: m) des verschmolzenen Teilbereichs darstellt und G ein Basisgewicht (Einheit: g/m²) darstellt,
**dadurch gekennzeichnet, dass** T von $5 \times 10^{-6}$ m bis $28 \times 10^{-6}$ m beträgt.

**2.** Mehrlagiger Gegenstand nach Anspruch 1, wobei der Index A von $0{,}20 \times 10^{-6}$ m³/g bis $0{,}72 \times 10^{-6}$ m³/g beträgt.

**3.** Mehrlagiger Gegenstand nach Anspruch 1, wobei das G 50 g/m² oder weniger beträgt.

**4.** Mehrlagiger Gegenstand nach Anspruch 3, wobei das G 48 g/m² oder weniger beträgt.

**5.** Mehrlagiger Gegenstand nach Anspruch 1, wobei der verschmolzene Teilbereich (100) einen verschmolzenen Teilbereich von Faserstücken enthält.

**6.** Mehrlagiger Gegenstand nach Anspruch 1, wobei eine Anzahl von Löchern mit jeweils einem Durchmesser von 100 $\mu$m oder mehr, die in dem verschmolzenen Teilbereich vorhanden sind, 10 Löcher/m² oder weniger beträgt.

**7.** Mehrlagiger Gegenstand nach Anspruch 1, wobei der verschmolzene Teilbereich ein geprägtes Muster aufweist.

**8.** Mehrlagiger Gegenstand nach Anspruch 1, wobei der mehrlagige Gegenstand ein weibliches Klettverschlusselement umfasst.

**9.** Mehrlagiger Gegenstand nach Anspruch 8, wobei der mehrlagige Gegenstand umfasst:

eine Eingriffsschicht (300), die mit einem männlichen Klettverschlusselement in Eingriff gebracht werden kann; und
eine Schicht mit physikalischen Eigenschaften (400), die gestaltet ist, um die Eingriffsschicht zu halten.

**10.** Mehrlagiger Gegenstand nach Anspruch 9, wobei eine Summe aus einem Basisgewicht eines Vliesstoffs in der Eingriffsschicht und einem Basisgewicht eines Vliesstoffs in der Schicht mit physikalischen Eigenschaften 60 g/m² oder weniger beträgt.

**11.** Mehrlagiger Gegenstand nach Anspruch 9, wobei eine Oberfläche einer Faser, die einen in der Eingriffsschicht enthaltenen Vliesstoff bildet, und eine Oberfläche der Schicht mit physikalischen Eigenschaften auf der Eingriffsschichtseite die gleiche Art von Polymer enthalten.

**12.** Klettverschluss, umfassend:

den mehrlagigen Gegenstand nach Anspruch 8; und
ein männliches Klettverschlusselement, das gestaltet ist, um mit dem mehrlagigen Gegenstand in Eingriff zu kommen.

**Revendications**

1. Article en couches, comprenant au moins une couche incluant un tissu non tissé de fibre, dans lequel :

   l'article en couches présente une partie fondue (100) ; et
   l'article en couches présente un indice A, lequel est représenté par l'équation (1), de 0,75x10$^{-6}$ m$^3$/g ou moins :

$$A=T/G \qquad (1)$$

   où T représente l'épaisseur (unité : m) de la partie fondue
   et G représente un poids de base (unité: g/m$^2$), **caractérisé en ce que**
   T va de 5x10$^{-6}$ m à 28x10$^{-6}$ m.

2. Article en couches selon la revendication 1, dans lequel l'indice A va de 0,20x10$^{-6}$ m$^3$/g à 0,72x10$^{-6}$ m$^3$/g.

3. Article en couches selon la revendication 1, dans lequel G vaut 50 g/m$^2$ ou moins.

4. Article en couches selon la revendication 3, dans lequel G vaut 48 g/m$^2$ ou moins.

5. Article en couches selon la revendication 1, dans lequel la partie fondue (100) inclut une partie fondues de morceaux de fibre.

6. Article en couches selon la revendication 1, dans lequel un nombre de trous présentant chacun un diamètre de 100 μm ou plus présent dans la partie fondue, est de 10 trous/m$^2$ ou moins.

7. Article en couches selon la revendication 1, dans lequel la partie fondue (100) présente un motif en reflief.

8. Article en couches selon la revendication 1, dans lequel l'article en couches comprend un élément femelle de fixation par crochets et boucles.

9. Article en couches selon la revendication 8, dans lequel l'article en couches comprend :

   une couche prenante (300) pouvant se prendre avec un élément mâle de fixation par crochets et boucles; et
   une couche de propriété physique (400) configurée pour maintenir la couche prenante.

10. Article en couches selon la revendication 9, dans lequel la somme d'un poids de base d'un tissu non tissé dans la couche prenante et d'un poids de base d'un tissu non tissé dans la couche de propriété physique, est de 60 g/m$^2$ ou moins.

11. Article en couches selon la revendication 9, dans lequel une surface de fibre formant un tissu non tissé inclus dans la couche prenante et la surface de la couche de propriété physique sur le côté couche prenante contiennent le même type de polymère.

12. Fixation par crochets et boucles, comprenant:

   l'article en couches selon la revendication 8 ; et
   un élément mâle de fixation par crochets et boucles réalisé pour se prendre avec l'article en couches.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H10280267 B
- JP 2009527315 A
- JP 2010125337 A
- JP 2012167412 A
- JP 2004176191 A
- JP 2014224330 A
- EP 3311687 A1
- EP 3311688 A1

- US 2006019572 A1
- JP 2011135985 A
- JP 2008526552 A
- JP 2010195044 A
- JP 2013231249 A
- JP 2015016294 A
- US 5976316 A

**Non-patent literature cited in the description**

- **E.A. VAUGHN.** Nonwoven Fabric Primer and Reference Sampler. Association of the Nonwoven Fabrics Industry, 1992